# FASCICULE DE BREVET EUROPEEN

(11) **EP 2 433 675 B1**
(45) Date de publication et mention de la délivrance du brevet: **09.01.2013**
(21) Numéro de dépôt: 11178361.9
(22) Date de dépôt: 22.08.2011
(51) Int. Cl.: A61N 1/372, A61B 5/00, H04B 13/00

(54) **Dispositif médical implantable actif comprenant des moyens de communication sans fil via des impulsions électriques conduites par les tissus interstitiels du corps**
Aktive implantierbare medizinische Vorrichtung, die drahtlose Kommunikationsmittel umfasst, die über elektrische Impulse kommunizieren, die von den interstitiellen Geweben des Körpers weitergeleitet werden
Active implantable medical device including a means for wireless communication via electric pulses conducted by the interstitial tissue of the body

(30) Priorité: 24.09.2010 FR 1057691
(43) Date de publication de la demande: 28.03.2012
(73) Titulaire: Sorin CRM SAS, 92140 Clamart Cedex (FR)
(72) Inventeur: Ghildiyal, Ashutosh, 92140 Clamart (FR); Dal Molin, Renzo, 92320 Châtillon (FR)
(74) Mandataire: Dupuis-Latour, Dominique

(56) Documents cités:
- WO-A1-2010/042066
- US-A- 4 987 897
- US-A1- 2002 099 423
- US-A1- 2007 088 397
- US-A1- 2007 239 229

## Description

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les implants cochléaires, etc., ainsi que les dispositifs de mesure de pH ou encore d'impédance intracorporelle (telle que mesure d'impédance transpulmonaire ou d'impédance intracardiaque).

L'invention concerne plus particulièrement ceux de ces dispositifs qui mettent en oeuvre des capsules autonomes implantées et dépourvues de toute liaison physique à un dispositif principal implanté (tel qu'un boîtier de générateur d'impulsions de stimulation) ou non implanté (périphérique externe tel que programmateur ou dispositif de *monitoring* pour le suivi à distance du patient). La communication, conduite par les tissus interstitiels du corps, est alors du type dit "HBC" *(Human Body Communication,* communication par voie intracorporelle).

Ces capsules autonomes sont dénommées pour cette raison "capsules *leadless",* pour les distinguer des électrodes ou des capteurs disposés à l'extrémité distale d'une sonde *(lead),* cette sonde étant parcourue sur toute sa longueur par un ou plusieurs conducteurs reliant par voie galvanique l'électrode ou le capteur à un générateur connecté à l'extrémité opposée, proximale, de la sonde.

De telles capsules *leadless* sont par exemple décrites dans les US 2007/0088397 A1 et WO 2007/047681 A2 (Nanostim, Inc.) ou encore dans le US 2006/0136004 A1 (EBR Systems, Inc.).

Ces capsules *leadless* peuvent être notamment des capsules épicardiques, fixées à la paroi extérieure du coeur, ou bien des capsules endocavitaires, fixées à la paroi intérieure d'une cavité ventriculaire ou auriculaire. Leur fixation à la paroi cardiaque se fait habituellement au moyen d'une vis d'ancrage hélicoïdale saillante, prolongeant axialement le corps de la capsule et destinée à pénétrer dans le tissu cardiaque par vissage au site d'implantation.

Une telle capsule comprend des circuits de détection/stimulation pour recueillir des potentiels de dépolarisation du myocarde et/ou pour appliquer des impulsions de stimulation au site où est implantée la capsule. La capsule porte alors une électrode appropriée, qui peut être notamment constituée par une partie active de la vis d'ancrage. Elle peut également incorporer un ou plusieurs capteurs permettant de mesurer localement la valeur d'un paramètre tel que le niveau d'oxygène dans le sang, la pression cardiaque endocavitaire, l'accélération de la paroi cardiaque, l'accélération du patient come indicateur de l'activité etc. Bien entendu, pour permettre l'échange de données à distance, ces capsules incorporent des moyens émetteurs/récepteurs de communication sans fil.

L'invention n'est toutefois pas limitée à un type particulier de capsule, et elle est applicable indifféremment à tout type de capsule *leadless,* quelle que soit sa destination fonctionnelle.

Plusieurs techniques ont été proposées pour assurer la communication sans fil entre ces capsules autonomes et un dispositif distant destiné à centraliser les informations recueillies par les capsules et à leur envoyer si nécessaire des commandes appropriées. Ce dispositif peut être notamment un stimulateur, resynchroniseur ou défibrillateur implanté, un défibrillateur sous-cutané, ou un enregistreur longue durée.

Ainsi, le US 2006/0136004 A1 propose de transmettre les données par des ondes acoustiques se propageant à l'intérieur du corps. Cette technique est efficace et sans danger ; elle présente toutefois l'inconvénient de nécessiter une puissance d'émission relativement élevée compte tenu de l'atténuation des ondes acoustiques dans le corps, et ne permet qu'un débit de données relativement faible.

Le US 5 411 535 A propose une autre technique, basée sur l'utilisation d'ondes radiofréquence (RF). Ici encore, une puissance d'émission relativement importante est nécessaire, et l'atténuation de ces ondes par les tissus intracorporels est un obstacle important à leur propagation.

Une autre technique encore a été proposée par le US 4 987 897 A, mais il s'agit d'un échange de données avec un dispositif externe (programmateur), par voie transcutanée et non plus intracorporelle. Cette transmission est assurée à courte distance entre, d'une part, le boîtier d'un stimulateur implanté dans une poche sous-cutanée et, d'autre part, un programmateur externe disposé à proximité de ce générateur. Les courants circulent donc au travers de la peau dans une région très éloignée des endroits sensibles, notamment à distance du myocarde, ce qui évite tout risque de perturbation des ondes de dépolarisation naturelle ou stimulée de ce dernier.

Le US 2007/0088397 A1 propose par ailleurs d'utiliser les impulsions de stimulation produites par une capsule comme véhicule pour la transmission de données préalablement recueillies ou élaborées par la capsule. Pour cela, l'impulsion, au lieu de présenter une variation monotone de tension, est interrompue de manière contrôlée pendant des durées très brèves de manière à créer dans le profil de l'impulsion de très étroits créneaux dont la succession correspond à un codage binaire de l'information à transmettre.

Cette technique permet de profiter de l'énergie élevée des impulsions de stimulation pour s'affranchir des problèmes d'atténuation au sein des tissus interstitiels entre la capsule et le dispositif.

Elle présente cependant un certain nombre d'inconvénients parmi lesquels :
- limitation à l'émission de données par une capsule active générant des impulsions : en l'absence d'impulsions générées, il n'est possible de transmettre aucune donnée puisque l'impulsion est le véhicule de l'information ;
- limitation à une situation où la stimulation est permanente ; sinon impossibilité de transmettre en continu des données, par exemple des signaux électriques recueillis par la capsule ou des valeurs suivies par un capteur intégré à la capsule ;
- limitation à une communication unidirectionnelle, de la capsule active produisant l'impulsion vers le dispositif récepteur distant, mais non dans le sens inverse ;
- faible débit de données, limitée à quelques bits d'informations par impulsion, et impossibilité de transmettre des informations à une cadence plus élevée que celle des impulsions de stimulation.

Le US 2002/0099423 A1 décrit une technique de communication sans fil intracorporelle entre un dispositif médical implanté et un dispositif externe pourvu d'électrodes en contact avec la peau du patient. L'implant génère des trains d'impulsions électriques dont le niveau se situe au-dessous du seuil de stimulation et applique ces impulsions à des électrodes pour leur permettre de se propager jusqu'à la surface du corps du patient, où elle seront captées par les électrodes du dispositif externe puis décodées par ce dernier.

Cette technique présente plusieurs inconvénients, notamment une consommation relativement élevée et la très grande variabilité en fonction de la résistance de charge vue par l'implant entre ses électrodes d'émission des impulsions. En outre et surtout, même avec des impulsions biphasiques (comme le prévoit ce document), il existe un risque élevé que des charges résiduelles subsistent, du fait d'un équilibrage imparfait des charges positives et négatives générées par les impulsions. Ces charges résiduelles vont produire une polarisation au sein des tissus, créant de ce fait un risque pour le patient.

Pour ces raisons, cette technique n'est pas adaptée à une communication permanente entre dispositifs médicaux, notamment entre deux implants (ce qui suppose que les impulsions traversent des régions excitables du myocarde). Elle n'est d'ailleurs proposée que pour une communication entre un implant et un dispositif externe par voie transcutanée, hors zones dangereuses. D'autre part, s'agissant d'une communication brève et temporaire (le dispositif externe est par exemple utilisé pour relever de temps en temps le niveau de la pile de l'implant), une consommation assez élevée ne constitue pas un facteur critique.

Le but de l'invention est de proposer une technique de communication sans fil par voie intracorporelle entre dispositifs médicaux implantables, typiquement entre une capsule *leadless* et un dispositif concentrateur implanté, par l'intermédiaire de signaux constitués d'impulsions électriques aptes à être conduites par les tissus interstitiels du corps, technique qui pallie les inconvénients précités et procure les avantages suivants :
- possibilité de communication par tout type de capsule *leadless,*
- innocuité totale pour le patient, même dans le cas de signaux transmis au sein même des tissus du myocarde ;
- technique ne nécessitant que peu d'énergie pour établir la communication, notamment compatible avec la relativement faible autonomie des capsules *leadless,* dépendantes d'un système d'auto-alimentation intégré ;
- débit de données élevé ;
- absence de risque de perturbations par des signaux électriques parasites présents au sein des tissus du corps, notamment de perturbations par les myopotentiels présents dans l'organisme.

A cet effet, l'invention propose un dispositif médical implantable actif du type général divulgué par le US 2002/0099423 A1 précité, c'est-à-dire comprenant des moyens pour communiquer sans fil par voie intracorporelle avec au moins un autre dispositif médical implantable actif par l'intermédiaire de signaux constitués d'impulsions électriques aptes à être conduites par les tissus interstitiels du corps. Ce dispositif comprend : au moins un couple d'électrodes ; des moyens générateurs, aptes à générer des trains d'impulsions formés d'une succession desdites impulsions électriques ; des moyens modulateurs, aptes à moduler les trains d'impulsions par des informations numériques produites par le dispositif, les impulsions étant des impulsions biphasiques comprenant une alternance positive et une alternance négative ; et des moyens pour injecter les impulsions entre les électrodes.

De façon caractéristique de l'invention, les impulsions biphasiques sont des impulsions de courant produites par une source de courant constant régulé comprise dans les moyens générateurs.

Selon diverses caractéristiques subsidiaires avantageuses :
- chaque alternance positive et négative de l'impulsion de courant biphasique est de forme carrée ;
- l'alternance négative de l'impulsion de courant biphasique suit l'alternance positive de cette même impulsion ou *vice versa ;*
- les alternances négative et positive de l'impulsion de courant biphasique sont des alternances symétriques ;
- les impulsions du train d'impulsions sont générées avec alternance de l'ordre des polarités des alternances d'une impulsion à la suivante, de sorte qu'une impulsion dont l'alternance positive précède l'alternance négative soit suivie d'une impulsion consécutive dont l'alternance négative précède l'alternance positive et *vice versa :* on peut ainsi avoir une impulsion positive (négative) suivie d'une impulsion négative (positive) suivie d'une impulsion négative (positive) suivie d'une impulsion positive (négative) ;
- les moyens modulateurs sont des moyens aptes à moduler l'intervalle temporel séparant des couples d'impulsions de courant biphasiques consécutives du trains d'impulsions, ou la largeur de ces impulsions, ou bien leur amplitude ;
- la durée de l'impulsion de courant biphasique est comprise entre 0,1 et 30 µs, de préférence 0,5 µs, la période de récurrence des impulsions de courant biphasiques est comprise entre 2 µs et 2 ms, de préférence 2 µs, et l'amplitude de chacune des alternances positive et négative de l'impulsion de courant est comprise entre 30 pA et 20 mA, de préférence 10 mA.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 illustre de façon schématique un ensemble de dispositifs médicaux comprenant notamment des capsules *leadless,* implantées au sein du corps d'un patient.
La Figure 2 montre plus précisément la manière d'implanter ces capsules *leadless* sur la paroi interne ou externe du myocarde.
La Figure 3 est un schéma par blocs fonctionnels montrant les différents étages constitutifs d'une capsule *leadless.*
La Figure 4 illustre les formes d'ondes permettant la communication sans fil par voie intracorporelle selon la technique de l'invention.
La Figure 5 illustre schématiquement les éléments côté émetteur et côté récepteur nécessaire à la mise en oeuvre de l'invention.
La Figure 6 illustre schématiquement un circuit permettant de générer les impulsions de courant servant à la communication intracorporelle.
La Figure 7 illustre les chronogrammes de séquencement des différents interrupteurs du circuit de la Figure 6.
La Figure 8 illustre le circuit démodulateur permettant de décoder les impulsions générées par le circuit émetteur.
La Figure 9 illustre des chronogrammes de signaux pris en différents endroits du démodulateur de la Figure 8 et expliquant le fonctionnement de celui-ci.

On va maintenant décrire un exemple de réalisation de l'invention.

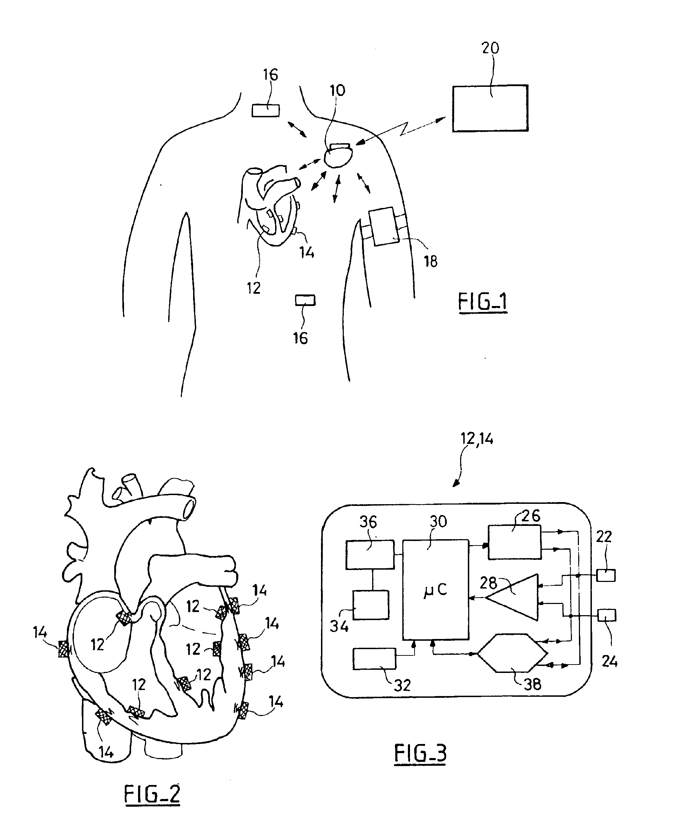

Sur la Figure 1 on a illustré un ensemble de dispositifs médicaux implantés au sein du corps d'un patient, communiquant entre eux sans fil par voie "HBC" *(Human Body Communication,* communication par voie intracorporelle).

Le patient est équipé par exemple d'un implant 10 tel qu'un défibrillateur/stimulateur/resynchroniseur implanté, ou un défibrillateur de type sous-cutané, ou encore un enregistreur longue durée. Ce dispositif implanté 10 est le dispositif maître d'un réseau comportant une pluralité de dispositifs esclaves 12 à 18 avec lesquels il est susceptible d'entrer en communication par voie HBC. Ces dispositifs peuvent notamment inclure des capsules intracardiaques 12 ou épicardiques 14 implantées directement sur le coeur du patient, d'autres dispositifs 16 tels que capteurs de myopotentiels ou dispositifs de stimulation neurologique, et éventuellement un dispositif externe 18 disposé sur un brassard et pourvu d'électrodes en contact avec la peau. Le dispositif 10 peut également être utilisé en tant que passerelle avec le monde extérieur pour communiquer avec un périphérique externe 20 du type programmateur ou dispositif de télétransmission de données avec lequel il pourra communiquer notamment par télémétrie RF dans la bande MICS *(Medical Implants Communication System)* 402-405 MHz, ou les bandes banalisées publiques ISM (Industriel, Scientifique et Médical) 863-870 MHz, 902-928 MHz et 2,4 GHz utilisées par les dispositifs médicaux.

Chacun des dispositifs 10 à 18 est muni d'au moins un couple d'électrodes qui se trouvent en contact direct avec les tissus du corps pour les dispositifs implantés, ou en contact avec la peau pour le dispositif externe 18.

Sur la Figure 2, on a représenté un exemple de capsules de type *leadless* implantées soit sur la partie antérieure du myocarde, à l'intérieur d'une cavité auriculaire ou ventriculaire (capsules endocavitaires 12), soit sur une paroi externe de ce même myocarde (capsules épicardiques 14). Ces capsules, qui sont par exemple décrites dans les US 2007/0088397 A1, WO 2007/047681 A2 et US 2006/0136004 A1 précités, sont fixées à la paroi cardiaque au moyen d'une vis d'ancrage saillante destinée à pénétrer dans le tissu cardiaque par vissage au site d'implantation. La vis peut être soit une vis passive, ne servant qu'à la fixation de la capsule, soit une vis active, servant à recueillir les signaux de dépolarisation se propageant dans les tissus du myocarde et/ou à délivrer des impulsions de stimulation au site d'implantation, de façon localisée.

La Figure 3 illustre de façon schématique les différents circuits internes des capsules 12, 14 (et, *mutatis mutandis,* des autres éléments implantés prévus pour communiquer entre eux par la technique de l'invention). Chaque capsule comporte un couple d'électrodes 22, 24, l'une d'entre elles pouvant d'ailleurs être constituée par la vis d'ancrage dans le tissu du coeur. Ces électrodes sont reliées à un circuit 26 générateur d'impulsions de stimulation (pour une capsule active incorporant cette fonction) et/ou à un circuit de détection 28 servant au recueil des potentiels de dépolarisation recueillis entre les électrodes 22 et 24. Un circuit central 30 assure le pilotage des différentes fonctions, la mémorisation des signaux recueillis, etc. La capsule peut également être pourvue d'un capteur 32 tel qu'un capteur d'accélération, de pression, un capteur hémodynamique, de température, de saturation en oxygène, etc. La capsule est alimentée par une petite batterie ou un circuit de récupération d'énergie 34 alimentant l'ensemble des circuits via un étage de gestion d'énergie 36.

De façon caractéristique de l'invention, les électrodes 22 et 24 sont, dans tous les cas, également reliées à un circuit modulateur/démodulateur 38 couplé au circuit processeur central 30 et apte à émettre et/ou recevoir des impulsions servant à la communication sans fil HBC, ces impulsions présentant des caractéristiques propres à l'invention, que l'on décrira plus bas.

Selon que les circuits de stimulation (module 26) et de recueil (module 28) sont présents ou non, les électrodes 22, 24 peuvent assurer une simple, double ou triple fonction, à savoir : stimulation et/ou recueil des potentiels cardiaques (le cas échéant) ; et/ou transmission des informations suivies par le capteur 32 (le cas échéant) ; et émission/réception pour la communication HBC (en tout état de cause).

Le circuit 30 inclut l'ensemble de l'électronique permettant de contrôler les diverses fonctions de la capsule. Il comprend un microcontrôleur et un oscillateur générant les signaux d'horloge nécessaires au fonctionnement du microcontrôleur et à la communication. Il peut également contenir un convertisseur analogique/numérique et une mémoire de stockage numérique.

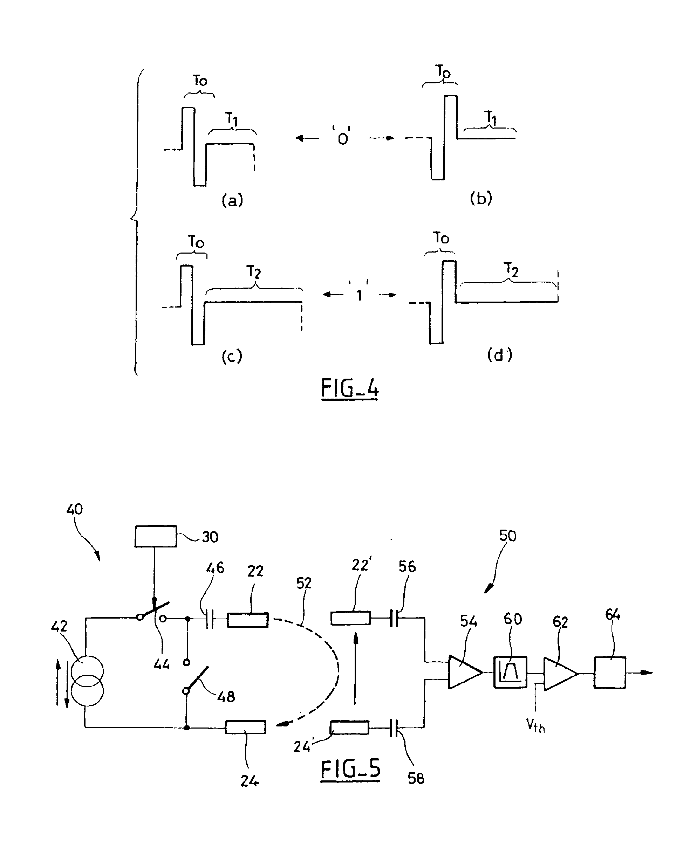

La Figure 4 illustre un exemple d'impulsion produite par le circuit 38 pour assurer la communication HBC au moyen d'impulsions électriques conduites par les tissus interstitiels du corps.

De façon caractéristique de l'invention, (i) ces impulsions sont des impulsions de courant, et (ii) chaque impulsion générée est une impulsion biphasique, afin de réduire au maximum les charges résiduelles injectées dans le coeur ou réduire la corrosion des matériaux.

Dans les exemples illustré Figure 4, ces impulsions comprennent deux alternances successives, positive et négative, de formes carrées et symétriques (même amplitude en valeur absolue, même durée pour les deux alternances). D'autres formes d'onde sont cependant envisageables, et l'exemple exposé ici n'est pas limitatif.

Toujours dans cet exemple, la modulation des impulsions résulte de l'intervalle temporel variable séparant des couples d'impulsions de courant biphasiques consécutives d'un train d'impulsions généré par le dispositif. Chaque impulsion est définie par la succession, de durée constante T₀, de deux alternances de signe opposé. Elle est suivie cette impulsion d'une attente respectivement courte T₁, pour coder par exemple un '0' binaire, ou longue T₂, pour coder un '1' binaire.

D'autres types de modulation sont toutefois envisageables, par exemple une modulation de l'amplitude des impulsions, de la largeur des alternances (modulation du type PWM), au lieu de la modulation ici décrite consistant à moduler l'intervalle temporel T₁ ou T₂ séparant des couples d'impulsions de courant consécutives d'un train d'impulsions donné.

L'impulsion biphasique peut être constituée d'une alternance positive suivie d'une alternance négative (Figures 4a et 4c), ou bien d'une alternance négative suivie d'une alternance positive (Figures 4b et 4d). Avantageusement, pour réduire au maximum les charges résiduelles qui pourraient résulter des imperfections d'une impulsion biphasique non exactement symétrique, après avoir émis une impulsion d'un premier type (par exemple alternance positive puis alternance négative, comme sur les Figures 4a ou 4c), l'impulsion suivante pourra être une impulsion de type inverse (alternance négative puis alternance positive, comme sur les Figures 4b ou 4d), ou non inverse.

Ceci permettra de compenser exactement l'injection éventuelle de charges résiduelles à chaque bit d'information envoyé, en respectant donc les normes médicales et en assurant l'innocuité des impulsions émises.

On pourra en outre mettre en place côté récepteur un contrôle d'erreur par vérification de la présence systématique de ce motif alterné au sein du train d'impulsions reçue.

Les impulsions biphasiques sont émises en succession sous forme de trains d'impulsions à une fréquence relativement élevée, typiquement à une cadence d'un bit toutes les 2 µs. La durée T₀ de l'impulsion est de l'ordre de 1000 ns, valeur qui s'avère adaptée à une transmission efficace au sein du corps humain.

Le choix d'une fréquence de répétition de l'ordre de 500 kHz (1 bit toutes les 2 µs en moyenne) permet d'adapter le contenu spectral au canal de transmission particulier constitué par les tissus interstitiels du corps, qui présente une atténuation minimale relativement modérée dans la bande 500 kHz-10 MHz (bande B). De façon générale, l'atténuation dans cette bande de fréquences varie entre 10 dB et 40 dB, selon la distance entre émetteur et récepteur, l'écartement entre les électrodes respectives du couple d'électrodes et la surface de ces électrodes, avec une valeur typique de l'ordre de 20 dB à 1 MHz sur une distance de 10 à 12 cm entre émetteur et récepteur.

La Figure 5 illustre schématiquement les moyens utilisés pour émettre et recevoir les impulsions biphasiques que l'on vient de décrire.

Les circuits de l'émetteur 40 se trouvent dans le stimulateur *leadless,* ou dans l'appareil sous-cutané, chargé de surveiller l'activité cardiaque et de générer des impulsions de stimulation, de resynchronisation, de défibrillation et/ou de cardioversion. Ils comprennent une source 42 de courant constant de l'ordre de 10 mA, ajustable périodiquement ou sur commande en fonction de la résistance de la sonde connectée au coeur pour générer en fin d'impulsion une tension de 2 V par exemple. Le module de commande 30 commande l'ouverture et la fermeture d'interrupteurs, en particulier la fermeture de l'interrupteur 44 afin d'injecter le courant sur un intervalle de temps prédéterminé, par exemple de l'ordre de 0,5 µs. Le courant injecté 52 circulera (via le condensateur de liaison 46, partagé ou non avec l'étage de stimulation, permettant d'éviter tout envoi de tension continue sur les électrodes) au travers du corps du patient depuis l'une des électrodes 22 jusqu'à l'autre électrode 24. L'interrupteur 48 permet de décharger ensuite le condensateur 46 de la charge résiduelle due aux erreurs de compensation des impulsions positives et négatives. Après avoir ainsi injecté une première alternance, on procède de même pour injecter l'alternance suivante en inversant le sens du courant, pour obtenir une impulsion du type de celles illustrées Figure 4.

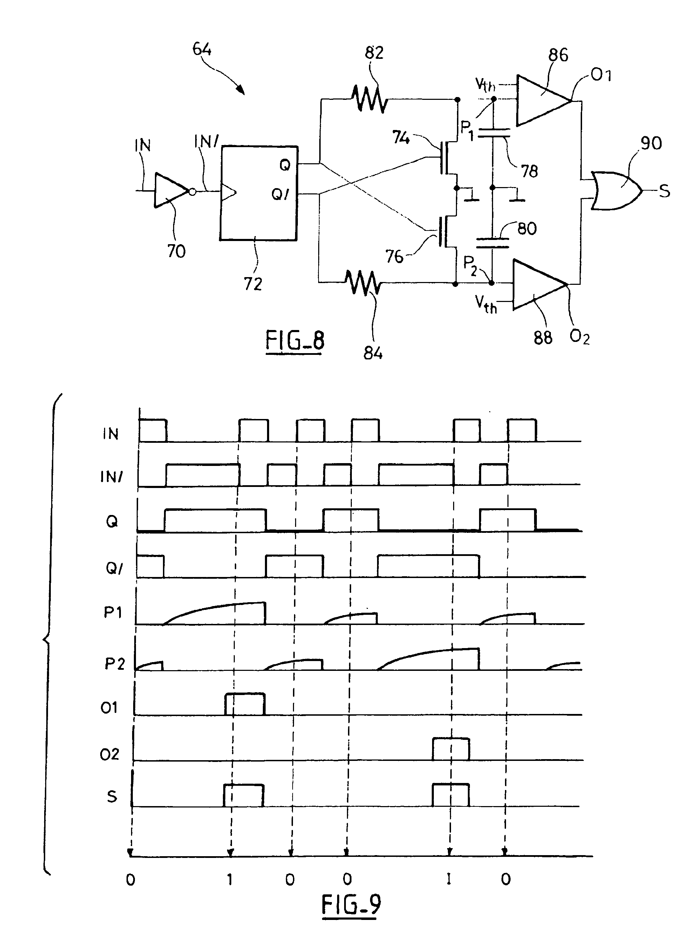

La référence 50 désigne les circuits utilisés du côté récepteur. Le courant 52 circulant dans le corps génère entre les électrodes 22' et 24' du récepteur une différence de potentiel qui est appliquée à un étage amplificateur 54 via les condensateurs de liaison 56 et 58 permettant d'éliminer toute composante continue. L'amplificateur ne sera alimenté que durant les périodes où des données sont obtenues, afin de réduire la consommation en courant. Le signal amplifié résultant est appliqué à un filtre passe-bande 60 afin de filtrer les signaux parasites hors de la bande pertinente. Le signal filtré obtenu est appliqué à un comparateur à seuil 62 et à un étage démodulateur 64 (ces circuits seront décrits plus en détail en référence aux Figures 8 et 9).

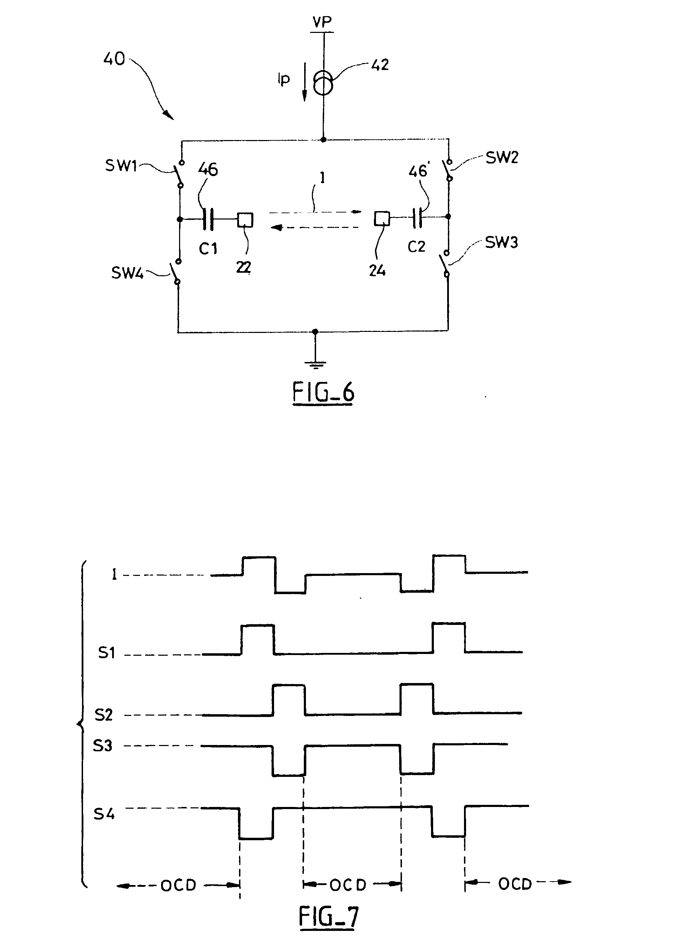

Les Figures 6 et 7 illustrent un schéma de principe de générateur de courant biphasique. La source de courant constant 42 est reliée aux électrodes 22 et 24 par deux interrupteurs SW₁ et SW₂ et par les condensateurs de liaison 46 et 46', eux-mêmes reliés à la masse par l'intermédiaire de deux interrupteurs SW₃ et SW₄. La Figure 7 donne les chronogrammes des différents signaux de commande S₁ à S₄ appliqués respectivement aux interrupteurs SW₁ à SW₄ (l'interrupteur étant fermé lorsque le signal est à l'état haut). La figure 7 indique également le profil du courant I circulant entre les deux électrodes 22 et 24. Sur ce chronogramme, on a ainsi représenté deux impulsions biphasiques successives, symétrisées, avec inversion de la séquence d'alternances (positive-puis-négative, négative-puis-positive) d'une impulsion à la suivante. Enfin, la période référencée OCD correspond à la décharge des condensateurs de sortie, qui est opérée par l'intermédiaire des interrupteurs SW₃ et SW₄. Dans le cas ou les condensateurs 46 et 46' sont partagés avec l'étage de stimulation, la décharge OCD se fera en période réfractaire seulement, du fait de sa durée plus longue.

La Figure 8 décrit un exemple de démodulateur permettant de décoder des impulsions telles que celles illustrées Figure 4, c'est-à-dire dont le codage binaire '0' ou '1' correspond à un temps d'attente respectivement court T₁ ou long T₂ d'une impulsion à la suivante. Ce démodulateur 64 reçoit les données du comparateur 62. Les données IN sont ensuite appliquées à un inverseur 70 dont la sortie IN/ commande une bascule bistable 72 dont la sortie Q et la sortie complémentaire Q/ sont reliés à la grille de transistors symétriques respectifs 74 et 76. Chacun de ces transistors charge un condensateur 78 ou 80 par une résistance 82 ou 84. La tension au point commun P₁ ou P₂ de chaque ensemble condensateur-résistance est appliqué à l'entrée d'un circuit comparateur respectif 86 ou 88 dont l'autre entrée est reliée à une tension de référence de seuil Vₜₕ. Les sorties O₁ et O₂ des comparateurs 86 et 88 sont appliquées à un circuit OU 90 dont la sortie S est telle que lorsque la charge de l'un ou l'autre condensateur 78 ou 80 atteint la valeur de seuil Vₜₕ, le démodulateur 64 émet une impulsion de sortie O, qui passera à zéro avec le front descendant du signal d'entrée IN.

La dernière ligne de la Figure 9 montra la manière dont on peut ainsi décoder un mot binaire '010010'.

## Revendications

1. Un dispositif médical implantable actif, comprenant des moyens pour communiquer sans fil par voie intracorporelle avec au moins un autre dispositif médical implantable actif par l'intermédiaire de signaux constitués d'impulsions électriques aptes à être conduites par les tissus interstitiels du corps, ce dispositif (12, 14) comprenant :
- au moins un couple d'électrodes (22, 24) ;
- des moyens générateurs, aptes à générer des trains d'impulsions formés d'une succession desdites impulsions électriques ;
- des moyens modulateurs, aptes à moduler les trains d'impulsions par des informations numériques produites par le dispositif,
les impulsions étant des impulsions biphasiques comprenant une alternance positive et une alternance négative ; et
- des moyens pour injecter les impulsions entre les électrodes (22, 24), dispositif **caractérisé en ce que** lesdites impulsions biphasiques sont des impulsions de courant produites par une source de courant constant régulé (42) comprise dans les moyens générateurs (44, 48).

2. Le dispositif de la revendication 1, dans lequel chaque alternance positive et négative de l'impulsion de courant biphasique est de forme carrée.

3. Le dispositif de la revendication 1, dans lequel l'alternance négative de l'impulsion de courant biphasique suit l'alternance positive de cette même impulsion, ou *vice versa.*

4. Le dispositif de la revendication 1, dans lequel les alternances négative et positive de l'impulsion de courant biphasique sont des alternances symétriques.

5. Le dispositif de la revendication 1, dans lequel les impulsions du train d'impulsions sont générées avec alternance de l'ordre des polarités des alternances d'une impulsion à la suivante, de sorte qu'une impulsion dont l'alternance positive précède l'alternance négative soit suivie d'une impulsion consécutive dont l'alternance négative précède l'alternance positive, et *vice versa.*

6. Le dispositif de la revendication 1, dans lequel les moyens modulateurs sont des moyens aptes à moduler l'intervalle temporel (T₁, T₂) séparant des couples d'impulsions de courant biphasiques consécutives du train d'impulsions.

7. Le dispositif de la revendication 1, dans lequel les moyens modulateurs sont des moyens aptes à moduler la largeur des impulsions de courant biphasiques successives du train d'impulsions.

8. Le dispositif de la revendication 1, dans lequel les moyens modulateurs sont des moyens aptes à moduler l'amplitude des impulsions de courant biphasiques successives du train d'impulsions.

9. Le dispositif de la revendication 1, dans lequel la durée (T₀) de l'impulsion de courant biphasique est comprise entre 0,1 et 30 µs, de préférence 0,5 µs.

10. Le dispositif de la revendication 1, dans lequel la période de récurrence des impulsions de courant biphasiques est comprise entre 2 µs et 2 ms, de préférence 2 µs.

11. Le dispositif de la revendication 1, dans lequel l'amplitude de chacune des alternances positive et négative de l'impulsion de courant est comprise entre 30 µA et 20 mA, de préférence 10 mA.

## Claims

1. Active implantable medical device, comprising means for wirelessly communicating by intracorporeal pathway with at least one other active implantable medical device via signals consisting of electrical pulses which can be conducted by the interstitial tissues of the body, this device (12, 14) comprising:
- at least one pair of electrodes (22, 24);
- generator means, capable of generating pulse trains formed by a succession of said electrical pulses;
- modulator means, which can modulate the pulse trains by digital information produced by the device,
the pulses being biphasic pulses comprising a positive alternation and a negative alternation; and
- means for injecting the pulses between the electrodes (22, 24),
the device being **characterized in that** said biphasic pulses are current pulses produced by a regulated constant current source (42) included in the generator means (44, 48).

2. Device of Claim 1, in which each positive and negative alternation of the biphasic current pulse is of square form.

3. Device of Claim 1, in which the negative alternation of the biphasic current pulse follows the positive alternation of this same pulse, or *vice versa.*

4. Device of Claim 1, in which the negative and positive alternations of the biphasic current pulse are symmetrical alternations.

5. Device of Claim 1, in which the pulses of the pulse train are generated with alternation of the order of the polarities of the alternations from one pulse to the next, so that a pulse whose positive alternation precedes the negative alternation is followed by a consecutive pulse whose negative alternation precedes the positive alternation, and *vice versa.*

6. Device of Claim 1, in which the modulator means are means which can modulate the time interval (T₁, T₂) separating pairs of consecutive biphasic current pulses of the pulse train.

7. Device of Claim 1, in which the modulator means are means which can modulate the width of the successive biphasic current pulses of the pulse train.

8. Device of Claim 1, in which the modulator means are means which can modulate the amplitude of the successive biphasic current pulses of the pulse train.

9. Device of Claim 1, in which the duration (T₀) of the biphasic current pulse is between 0.1 and 30 µs, preferably 0.5 µs.

10. Device of Claim 1, in which the period of recurrence of the biphasic current pulses is between 2 µs and 2 ms, preferably 2 µs.

11. Device of Claim 1, in which the amplitude of each of the positive and negative alternations of the current pulse is between 30 µA and 20 mA, preferably 10 mA.

## Patentansprüche

1. Aktive implantierbare medizinische Vorrichtung, die Einrichtungen enthält, um drahtlos auf intrakorporalem Weg mit mindestens einer anderen aktiven implantierbaren medizinischen Vorrichtung mittels Signalen zu kommunizieren, die aus elektrischen Impulsen bestehen, welche von den interstitiellen Geweben des Körpers geleitet werden können, wobei diese Vorrichtung (12, 14) Folgendes enthält:
- mindestens ein Paar von Elektroden (22, 24);
- Generatoreinrichtungen, die Impulsreihen erzeugen können, welche von einer Folge der elektrischen Impulse geformt werden;
- Modulatoreinrichtungen, die die Impulsreihen durch digitale Informationen modulieren können, die von der Vorrichtung erzeugt werden,
wobei die Impulse Zweiphasen-Impulse sind, die eine positive Halbperiode und eine negative Halbperiode enthalten; und
- Einrichtungen, um die Impulse zwischen die Elektroden (22, 24) einzuspeisen,
wobei die Vorrichtung **dadurch gekennzeichnet ist, dass** die Zweiphasen-Impulse Stromimpulse sind, die von einer Quelle geregelten Konstantstroms (42) erzeugt werden, die in den Generatoreinrichtungen (44, 48) enthalten ist.

2. Vorrichtung nach Anspruch 1, wobei jede positive und negative Halbperiode des Zweiphasen-Stromimpulses rechteckig ist.

3. Vorrichtung nach Anspruch 1, wobei die negative Halbperiode des Zweiphasen-Stromimpulses auf die positive Halbperiode dieses gleichen Impulses folgt oder umgekehrt.

4. Vorrichtung nach Anspruch 1, wobei die negative und die positive Halbperiode des Zweiphasen-Stromimpulses symmetrische Halbperioden sind.

5. Vorrichtung nach Anspruch 1, wobei die Impulse der Impulsreihe mit Halbperiode der Reihenfolge der Polaritäten der Halbperioden von einem Impuls zum anderen erzeugt werden, so dass auf einen Impuls, dessen positive Halbperiode vor der negativen Halbperiode liegt, ein nachfolgender Impuls folgt, dessen negative Halbperiode vor der positiven Halbperiode liegt, und umgekehrt.

6. Vorrichtung nach Anspruch 1, wobei die Modulatoreinrichtungen Einrichtungen sind, die das Zeitintervall (T₁, T₂) modulieren können, das aufeinanderfolgende Zweiphasen-Stromimpulspaare der Impulsreihe trennt.

7. Vorrichtung nach Anspruch 1, wobei die Modulatoreinrichtungen Einrichtungen sind, die die Breite der aufeinanderfolgenden Zweiphasen-Stromimpulse der Impulsfolge modulieren können.

8. Vorrichtung nach Anspruch 1, wobei die Modulatoreinrichtungen Einrichtungen sind, die die Amplitude der aufeinanderfolgenden Zweiphasen-Stromimpulse der Impulsreihe modulieren können.

9. Vorrichtung nach Anspruch 1, wobei die Dauer (T₀) des Zweiphasen-Stromimpulses zwischen 0,1 und 30 µs, vorzugsweise bei 0,5 µs liegt.

10. Vorrichtung nach Anspruch 1, wobei die Wiederholungsperiode der Zweiphasen-Stromimpulse zwischen 2 µs und 2 ms, vorzugsweise bei 2 µs liegt.

11. Vorrichtung nach Anspruch 1, wobei die Amplitude jeder der positiven und negativen Halbperioden des Stromimpulses zwischen 30 pA und 20 mA, vorzugsweise bei 10 mA liegt.
